**Europäisches Patentamt**

**European Patent Office**

**Office européen des brevets**

(19)

(11) Veröffentlichungsnummer: **0 090 339**
A2

(12) # EUROPÄISCHE PATENTANMELDUNG

(21) Anmeldenummer: **83102863.4**

(22) Anmeldetag: **23.03.83**

(51) Int. Cl.³: **C 12 Q 1/00**

(30) Priorität: **27.03.82 DE 3211347**

(43) Veröffentlichungstag der Anmeldung: **05.10.83**
**Patentblatt 83/40**

(84) Benannte Vertragsstaaten: **AT BE CH DE FR GB IT LI LU NL SE**

(71) Anmelder: **Boehringer Mannheim GmbH,**
**Sandhoferstrasse 116, D-6800 Mannheim 31 (DE)**

(72) Erfinder: **Beutler, Hans-Otto, Dr.rer.nat.,**
**Zugspitzstrasse 28, D-8132 Tutzing (DE)**
Erfinder: **Möller, Gerald, Dr.rer.nat.,**
**Waxensteinstrasse 17, D-8132 Tutzing (DE)**
Erfinder: **Schiefer, Sigbert, Dr.rer.nat., Moosstrasse 3,**
**D-8121 Pähl (DE)**

(54) **Verfahren und Reagens zur enzymatischen Bestimmung von Phosphatidylglycerin.**

(57) Mit Phospholipase-D mikrobieller Herkunft, insbesondere aus Streptomyces chromofuscus, gelingt es, Phosphatidylglycerin quantitativ in Phosphatidsäure und Glycerin zu spalten. Diese enzymatische Reaktion läßt sich zur Bestimmung des Phosphatidylglyceringehaltes in Flüssigkeiten, insbesondere im Fruchtwasser, heranziehen. Die Erfindung betrifft somit ein Verfahren zur enzymatischen Bestimmung von Phosphatidylglycerin, bei dem Phosphatidylglycerin mit Hilfe von mikrobieller Phospholipase-D in Phosphatidsäure und Glycerin und das freie Glycerin in bekannter Weise bestimmt wird. Ferner wird ein Reagens, das eine mikrobielle Phospholipase-D enthält, zur Durchführung des Verfahrens zur Bestimmung von Phosphatidglycerin, beansprucht.

0090339

Verfahren und Reagens zur enzymatischen Bestimmung
von Phosphatidylglycerin

_____

Zur Beurteilung der Lungenreife eines Föten werden im
Fruchtwasser üblicherweise das Lecithin/Sphingomyelin-Verhältnis und auch das Lecithin quantitativ bestimmt. In manchen
Fällen sind diese Meßdaten jedoch unzuverlässig oder
zumindest unvollständig. Vor allem, wenn komplikative
Situationen zu erwarten sind, wie beispielsweise bei
schwangeren Diabetikerinnen, beim Vorliegen eines Hydramnions oder bei anderen klinisch unklar zu beurteilenden
Schwangerschaftsphasen, ist die Risikobeurteilung einer
Entbindung anhand der üblichen Parameter: Lecithingehalt und L/S-Ratio nicht ausreichend. Es hat sich
gezeigt, daß in diesen Fällen die zusätzliche Bestimmung des Phosphatidylglycerin-Gehaltes die Beurteilung
der Lungenreife und damit verbunden der Gefahr eines
Atemnotsyndroms während der Entbindung mit einem
höheren Maß an Sicherheit zuläßt.

Die bisher bekannten Verfahren zur Messung von Phosphatidylglycerin sind sehr mühsam. Sie beruhen auf chromatographischen Methoden. Überwiegend sind es dünnschichtchromatographische Verfahren, mit denen Phosphatidylglycerin aus dem Fruchtwasser isoliert wird. Auch
säulenchromatographische Methoden werden angewendet.
Die dabei isolierten Phosphatide werden mit Hilfe üblicher Anfärbetechniken oder auch nach Verkohlung mit Schwefelsäure densitometrisch bestimmt. Beschrieben wird auch die Abtrennung des Phophatidylglycerins und die Unterscheidung von Begleitstoffen mit Hilfe einer zweidimensionalen Dünnschichtchromatographie.

Allen bekannten Methoden zur Bestimmung von Phosphatidylglycerin gemeinsam ist der hohe Zeitaufwand und die Erkenntnis, daß dabei hohe Verluste an Phosphatidylglycerin auftreten, die eine sichere Gehaltsbestimmung nicht zulassen.

Aufgabe der Erfindung war es, ein Verfahren bereitzustellen, mit dem Phosphatidylglycerin auf einfache, schnelle und zuverlässige Weise bestimmt werden kann.

Gelöst wird diese Aufgabe durch das erfindungsgemäße Verfahren, bei dem Phosphatidylglycerin mit mikrobieller Phospholipase-D enzymatisch in Phosphatidsäure und freies Glycerin gespalten und das freigesetzte Glycerin in bekannter Weise bestimmt wird. Gegenstand der Erfindung ist daher ein Verfahren zur enzymatischen Bestimmung von Phosphatidylglycerin, bei dem zunächst Phosphatidylglycerin mit Hilfe von Phospholipase-D zu Phosphatidsäure und Glycerin gespalten und das freie Glycerin in bekannter Weise bestimmt wird.

Es sind verschiedene Phospholipasen-D bekannt, beispielsweise von pflanzlicher Herkunft, zum Beispiel aus Weißkohl, oder von mikrobiellem Ursprung, zum Beispiel aus Streptomyces chromofuscus. Phospholipase-D spaltet in Phospholipiden üblicherweise die Bindung zwischen der Phosphatidsäure und dem Cholin bzw. anderen Stickstoffbasen, wie sie beispielsweise im Lecithin, Lysolecithin, Sphingomyelin und Kephalin vorliegen. In H.U. Bergmeyer, Methoden der enzymatischen Analyse, 3. Auflage, S. 1864, wird in dem Artikel über die Bestimmung von Lecithin im Abschnitt über die Spezifität dieser Nachweismethode dargelegt, daß die Phospholipase-D nur die Bindung zwischen Stickstoffbase und Phosphorsäure spaltet. Auch die ausführliche Bezeichnung für die Phospholipase-D: Phosphatidylcholinphosphatidohydrolase (vgl. a.a.O. S. 537) weist auf die spezifische Spaltung einer Phosphatidylcholin-Bindung hin. Tatsächlich gelang es auch nicht mit Hilfe der Phospholipase-D aus Weißkohl Phosphatidylglycerin, bei dem eine Phosphorsäure-Kohlenstoff-Bindung zu spalten ist, zu Phosphatidsäure und freiem Glycerin zu hydrolysieren.

Aus J.Biochem. 85 (1979) S. 79-95 ist eine Phospholipase-D mikrobiellen Ursprungs bekannt. Für dieses Enzym, das aus Streptomyces chromofuscus isoliert werden konnte, wird ebenfalls nur die Spaltung von Phosphorsäure-Stickstoff-Bindungen beschrieben. Überraschenderweise wurde jedoch festgestellt, daß mit Hilfe dieses Enzyms auch die Phosphorsäure-Kohlenstoff-Bindung im Phosphatidylglycerin quantitativ gespalten werden kann.

Die Bestimmung des freigesetzten Glycerins kann nach üblichen Methoden erfolgen, beispielsweise indem es durch Adenosintriphosphat und Glycerinkinase in Glycerophosphat übergeführt, letzteres mit Nicotinamid-adenindinucleotid in oxidierter Form und Glycerin-3-phosphat-Dehydrogenase dehydriert und die Zunahme an Nicotinamidadenin-dinucleotid in reduzierter Form gemessen wird. Besonders vorteilhaft ist es, nicht das bei der Umsetzung von Glycerin mit Adenosintriphosphat und Glycerinkinase entstehende Glycerophosphat, sondern das dabei ebenfalls gebildete Adenosindiphosphat mit bekannten Methoden nachzuweisen. Dies erfolgt zweckmäßig

0090339

durch Umsetzen mit Phosphoenolpyruvat und Pyruvatkinase, wobei Adenosintriphosphat rückgebildet wird und außerdem Pyruvat entsteht. Letzteres kann mit Nicotinamid-adenin-dinucleotid in reduzierter Form und Lactatdehydrogenase in Lactat überführt werden. Die Abnahme an Nicotinamid-adenin-dinucleotid in reduzierter Form wird gemessen.

Gemäß einer bevorzugten Ausführungsform des erfindungsgemäßen Verfahrens wird zunächst die Probe - beispielsweise Fruchtwasser, gegebenenfalls mit Wasser verdünnt - mit einem geeigneten Puffer-System, pH 7,0 bis 9,0 vorzugsweise Borat/Borax-Puffer, pH 7,0 bis 8,5 versetzt. Zu dieser Lösung wird ein Gemisch hinzugegeben, das Nicotinamid-adenin-dinucleotid in reduzierter Form, Adenosintriphosphat und Phosphoenolpyruvat enthält. Danach wird eine Enzymlösung mit Pyruvatkinase und Lactatdehydrogenase zugegeben und anschließend mit Glycerokinase-Lösung vermischt. Nach kurzer Wartezeit (ca. 5 Min.) wird die Extinktion $E_1$ gemessen. An-schließend wird durch Zugabe von mikrobieller Phospholipase-D die enzymatische Reaktionskette gestartet. Ist die Reaktion abgeschlossen (nach ca. 35-40 Min.) wird die Extinktion $E_2$ gemessen. Aus der Extinktions-differenz läßt sich der Phosphatidylglycerin-Gehalt der Probe ermitteln. Es empfiehlt sich, neben der eigentlichen Bestimmung der Probe einen Reagentienleerwert zu messen. Es ist zweckmäßig, die für das erfindungsgemäße Verfahren benötigten Enzyme und Hilfsstoffe zu bestimmten Lösungen bzw. Suspensionen zusammenzufassen. Als Puffer-System wird vorzugsweise ein Borat/Borax-Puffer mit pH 7,0-8,5 in einer Konzentration von 0,05-0,5 mol/l Pufferlösung, vorzugsweise von 0,07-0,25 Mol/l Pufferlösung, eingesetzt. Ganz besonders bevorzugt ist ein Borat/Borax-Puffer vom pH-Wert 7,8-8,2, der in einer Konzentration von
0,1-0,2 mol/1 Pufferlösung bei der Bestimmung benutzt
wird. NADH, ATP, Phosphoenolpyruvat und Glukose werden
vorteilhafterweise als "Coenzymlösung" zusammengefaßt.
Die einzelnen Konzentrationen in dieser Lösung betragen 2,5-6,0 mmol/1, vorzugsweise 4,0 mmol/1 NADH,
10-30 mmol/1, vorzugsweise 20 mmol/1 ATP und 10-60 mmol/1,
vorzugsweise 45 mmol/1 Phosphoenolpyruvat .

Pyruvatkinase und Lactatdehydrogenase werden zusammen
als "Enzymsuspension" zugegeben, wobei die Konzentration
an Pyruvatkinase und an Lactatdehydrogenase jeweils
150-1000 U/ml, vorzugsweise 300 U/ml betragen.

Glycerokinase wird vorzugsweise in Form einer getrennten
Lösung mit einem Gehalt an 50-200 U/ml, vorzugsweise 85 U/ml
verwendet.

Als mikrobielle Phospholipase-D wird vorzugsweise die
Phospholipase-D aus Streptomyces chromofuscus eingesetzt und zwar in Form einer wäßrigen Lösung, die
50-300 U/ml, vorzugsweise 100-200 U/ml Enzym enthält.

Gegenstand der Erfindung ist ferner ein Reagens zur
enzymatischen Bestimmung von Phosphatidylglycerin,
durch dessen Spaltung in Phosphatidsäure und Glycerin
und Bestimmen des freigesetzten Glycerins in bekannter
Weise. Das Reagens enthält die für die jeweilige
Methode zur Bestimmung des Glycerins üblicherweise erforderlichen Stoffe sowie mikrobielle Phospholipase-D,
vorzugsweise aus Streptomyces chromofuscus, zur Spaltung des Phosphatidylglycerins. Ein im Sinne der
Erfindung vorteilhaftes Reagens besteht aus folgenden
Bestandteilen:

0090339

a) einer Pufferlösung mit 0,0-0,5 mol/1 Borat/Borax-Puffer, pH 7,0-8,5

b) einer Coenzymlösung mit 2,5-6,0 mmol/1 NADH
10-30 mmol/1 ATP
10-60 mmol/1 Phosphoenolpyruvat

c) einer Enzymsuspension mit 150-1000 U/ml Pyruvatkinase
150-1000 U/ml Lactatdehydrogenase

d) einer Lösung mit 50-200  U/ml Glycerokinase

e) einer Lösung mit 50-300 U/ml mikrobielle Phospholipase-D.

Ganz besonders bevorzugt ist ein Reagens mit folgenden Bestandteilen:

a) einer Pufferlösung mit 0,02 mol/1 Borat/Borax-Puffer, pH 7,8-8,2

b) einer Coenzymlösung mit 4,0 mmol/1 NADH
20 mmol/1 ATP
45 mmol/1 Phosphoenolpyruvat

c) einer Enzymsuspension mit 300 U/ml Pyruvatkinase
   300 U/ml Lactatdehydrogenase

d) einer Lösung mit 85 U/ml Glycerokinase

e) einer Lösung mit 100-200 U/ml Phospholipase-D aus Streptomyces chromofuscus.

Die Erfindung wird durch das folgende Beispiel näher erläutert:

Beispiel

Bestimmung Phosphatidylglycerin in Fruchtwasser

Küvetten (Schichtdicke: 1 cm; Testvolumen: 2,13 ml; Meßwellenlänge: 365 mm (Hg)) werden bei 25$^o$C mit den folgenden Lösungen gefüllt:

0,10 ml einer Probelösung, die einen Phosphatidylglycerin-Gehalt von 2 mmol/1 oder weniger aufweist, sowie 1 ml einer Lösung, die 0,2 mol/1 Borat/Borax- Puffer, pH 7,8-8,2 enthält. Zu diesem Gemisch werden 0,1 ml einer Lösung aus 4,0 mmol/1 NADH, 20 mmol/1 ATP und 45 mmol/1 Phosphoenolpyruvat sowie 0,02 ml eines Enzymgemisches aus 300 U/ml Pyruvatkinase und 300 U/ml Lactatdehydrogenase zugegeben. Dieses Gemisch wird mit 0,01 ml einer Lösung, die 85 U/ml Glycerokinase enthält, vermischt. Die erhaltene Lösung wird durchmischt und 5 Min. bei Raumtemperatur stehengelassen. Die Extinktion $E_1$ wird gemessen. Die enzymatische Umsetzung wird gestartet durch Zusatz von 0,05 ml einer Phospholipase- D-Lösung mit ca. 150 U/ml. Nach ca. 35-40 Min. Stehenlassen bei Raumtemperatur wird die Extinktion $E_2$ ge- messen. Aus der Extinktionsdifferenz $E_1 - E_2$ wird die Extinktionsabnahme $\Delta$E berechnet, aus der sich der Phosphatidylglycerin-Gehalt der Probe nach der Formel:

$$c\underline{/\bar{g}/1\underline{/} = \Delta E \cdot 4,517$$

ergibt.

Zu jeder Bestimmung von Phosphatidylglycerin in einer Probelösung wird ein Reagentienleerwert ermittelt, der sich ergibt, wenn bei dem oben beschriebenen Bestimmungsverfahren anstelle der Probelösung destilliertes Wasser eingesetzt wird. Der Reagentienleerwert $\Delta$E wird vor der

0090339

Berechnung des Phosphatidylglycerin-Gehaltes nach der o.g. Formel von der gefundenen Extinktionsabnahme der Probe $\Delta E_p$ abgezogen.

## Patentansprüche

1. Verfahren zur enzymatischen Bestimmung von Phosphatidylglycerin, bei dem Phosphatidylglycerin mit Hilfe von mikrobieller Phospholipase-D in Phosphatidsäure und Glycerin gespalten und das freie Glycerin in bekannter Weise bestimmt wird.

2. Verfahren gemäß Anspruch 1, dadurch gekennzeichnet, daß Phospholipase-D aus Streptomyces chromofuscus eingesetzt wird.

3. Verfahren gemäß einem der Ansprüche 1 und 2, dadurch gekennzeichnet, daß das Glycerin bestimmt wird, indem es durch Adenosintriphosphat und Glycerokinase in Glycerophosphat übergeführt, letzteres mit Nicotinamid-adenin-dinucleotid in oxidierter Form und Glycerin-3-phosphat-Dehydrogenase dehydriert und die Zunahme an Nicotinamid-adenin-dinucleotid in reduzierter Form gemessen wird.

4. Verfahren gemäß einem der Ansprüche 1 und 2, dadurch gekennzeichnet, daß das Glycerin bestimmt wird, indem es durch Adenosintriphosphat und Glycerokinase in Glycerophosphat und Adenosindiphosphat übergeführt und letzteres nach bekannten Methoden bestimmt wird.

5. Reagens zur Durchführung des Verfahrens gemäß Anspruch 1, das eine mikrobielle Phospholipase-D enthält.

6. Reagens gemäß Anspruch 5, dadurch gekennzeichnet, daß Phospholipase-D aus Streptomyces chromofuscus enthalten ist.

7. Reagens gemäß Anspruch 5, gekennzeichnet durch folgende Bestandteile:

   a) einer Pufferlösung mit
      0,05-0,5 mol/1 Borat/Borax-Puffer, pH 7,0-8,5

   b) einer Coenzymlösung mit
      2,5-6,0 mmol/1 NADH
      10-30 mmol/1 ATP
      10-60 mmol/1 Phosphoenolpyruvat

   c) einer Enzymsuspension mit
      150-1000 U/ml Pyruvatkinase
      150-1000 U/ml Lactatdehydrogenase

   d) einer Lösung mit
      50-200 U/ml Glycerokinase

   e) einer Lösung mit
      50-300 U/ml mikrobielle Phospholipase-D.

8. Reagens gemäß Anspruch 6, gekennzeichnet durch folgende Bestandteile:

   a) einer Pufferlösung mit
      0,2 mol/1 Borat/Borax-Puffer, pH 7,8-8,2

   b) einer Coenzymlösung mit
      4,0 mmol/1 NADH
      20 mmol/1 ATP
      45 mmol/1 Phosphoenolpyruvat

   c) einer Enzymsuspension mit
      300 U/ml Pyruvatkinase
      300 U/ml Lactatdehydrogenase

0090339

d) einer Lösung mit 85 U/ml Glycerokinase

e) einer Lösung mit 100-200 U/ml Phospholipase-D aus Streptomyces chromofuscus.